Europäisches Patentamt

European Patent Office

Office européen des brevets

(11) Publication number: **0 196 858 B1**

# EUROPEAN PATENT SPECIFICATION

(45) Date of publication of patent specification: **18.09.91**  (51) Int. Cl.⁵: **C12N 1/20, A61K 35/74**

(21) Application number: **86302180.4**

(22) Date of filing: **25.03.86**

(54) Liver function-improving agent and blood pressure-lowering agent.

(30) Priority: **26.03.85 JP 59679/85**

(43) Date of publication of application:
**08.10.86 Bulletin 86/41**

(45) Publication of the grant of the patent:
**18.09.91 Bulletin 91/38**

(84) Designated Contracting States:
**CH DE FR GB IT LI NL SE**

(56) References cited:
**EP-A- 0 101 209**

(73) Proprietor: **KABUSHIKI KAISYA ADVANCE**
**5-7, Nihonbashi Kobuna-cho**
**Chuo-ku Tokyo 103(JP)**

(72) Inventor: **Kawai, Yasuo**
**2-8-12, Moridai**
**Atsugi-shi Kanagawa(JP)**
Inventor: **Ishihara, Kazuoki**
**2-13-7, Uchikanda Chiyoda-ku**
**Tokyo(JP)**
Inventor: **Yazawa, Kazunaga**
**Green-haitsu D 1-501 571, Unomori**
**Sagamihara-shi Kanagawa(JP)**

(74) Representative: **Ellis, John Clifford Holgate et al**
**MEWBURN ELLIS & CO. 2/3 Cursitor Street**
**London EC4A 1BQ(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

BACKGROUND OF THE INVENTION

1. Field of the Invention

The present invention relates to a new liver function-improving agent and a new blood pressure-reducing agent, processes for producing the same, and pharmaceutical preparations containing the same.

2. Description of the Related Art

Recently, the death rate from middle-aged or geriatric diseases has remarkably increased, and thus the prevention and therapy of hepatopathia and hypertention have become very important. Accordingly, the need for developing effective prevention and therapy of hepatopathia and hypertention has remarkably increased.

SUMMARY OF THE INVENTION

Accordingly, an object of the present invention is to provide novel bacterial cell products having liver function-improving activities, i.e., activities that will reduce the serum GOT and GPT levels, and having blood pressure-lowering activities in mammals.

Another and further objects of the present invention are to provide processes for preparing bacterial cell products having liver function-improving activities, i.e., activities that will reduce the serum GOT and GPT levels, and having blood pressure-lowering activities in mammals, pharmaceutical preparations containing the bacterial cell products, and methods for lowering the serum GOT and GPT levels and the blood pressure in mammals.

A still further object of the present invention is to provide a novel method to improve the liver function and to lower the blood pressure in mammals.

In accordance with the present invention, there is provided a process for preparing the bacterial cell products having activities that will reduce the serum GOT and GPT levels, and blood pressure-lowering activities in mammals comprising the steps of cultivating microorganisms belonging to the genus Streptococcus therefor, and collecting the cultivated microorganisms from the culture.

In accordance with the present invention, there is also provided a pharmaceutical composition for lowering the serum GOT and GPT levels, and blood pressure of patients, comprising a preventive or therapeutically effective amount of living cells, dead cells, or a mixture thereof, of microorganisms belonging to the genus Streptococcus and having liver function-improving and blood pressure-lowering activities in mammals, and a pharmaceutical carrier therefor. Such microorganisms can be found amongst the group consisting of Streptococcus faecium, S. faecalis, S. avium, S. durans, S. salivarius, S. mitis, and S. equinus.

In accordance with the present invention, there is still further provided a method for lowering the serum GOT and GPT levels, and the blood pressure of mammals, comprising orally administering to mammals an effective amount of living cells, dead cells, or a mixture thereof, of a microorganism belonging to the genus Streptococcus and having liver function-improving and blood pressure-lowering activities in mammals.

BRIEF DESCRIPTION OF THE DRAWING

The present invention will be better understood from the description set forth below with reference to the accompanying drawings, in which:

Fig. 1 illustrates the correlation between GOT values and the administration period in Example 1;

Fig. 2 illustrates the correlation between GPT values and the administration period in Example 1; and

Fig. 3 illustrates the correlation between the reduction rate (%) of the blood pressure and the administration period in Example 3.

DESCRIPTION OF THE PREFERRED EMBODIMENTS

The present inventors have found that various living cells and dead cells of microorganisms belonging to the genus Streptococcus can effectively reduce the serum GOT and GPT levels, and the blood pressure in mammals. These microorganisms derived from so-called gastrointestinal bacteria are substantially nontoxic when orally administered, and the microorganisms have an extremely high pharmacological

2

activity.

The types and the preparation methods of strains, and the pharmacological effects of the microorganisms according to the present invention will now be explained in detail.

Microorganisms

1. Species

Microorganisms utilizable in the present invention belonging to the genus Streptococcus: Streptococcus faecium, Streptococcus faecalis, Streptococcus avium, Streptococcus durans, Streptococcus salivarius, Streptococcus mitis, Streptococcus equinus, and others, are preferably shown.

Typical examples of such microorganisms have been deposited since July 15, 1982 in the Fermentation Research Institute (FRI) in Japan (all the numbers quoted as "FERM-P" in Table 1 refer to the deposition numbers of said Institute) and transferred to the Fermentation Research Institute (FRI) (i.e., International Depository Authority under Budapest Treaty in Japan) as the following FERM-BP deposition numbers in Table 1 under Budapest Treaty on the International Recognition of the Deposit of Microorganisms for the Purpose of Patent Procedure:

## Table 1

| Strains | Deposition number | Deposition number |
|---|---|---|
| Streptococcus faecium | FERM P-6624 | FERM BP-296 |
| Streptococcus faecalis | FERM P-6625 | FERM BP-297 |
| Streptococcus avium | FERM P-6626 | FERM BP-298 |
| Streptococcus salivarius | FERM P-6627 | FERM BP-299 |
| Streptococcus durans | FERM P-6628 | FERM BP-300 |
| Streptococcus mitis | FERM P-6629 | FERM BP-301 |
| Streptococcus equinus | FERM P-6630 | FERM BP-302 |

2. Microbiological Characteristics of Microorganisms

General Microbiological Characteristics

The microbiological characteristics of the microorganisms in the present invention are the same as those of known microorganisms belonging to the identical class. That is, the general microbiological characteristics, cultivation methods, and other properties correspond to those described in the following articles:
1) Bergey's Manual of Determinative Bacteriology, 8th ed., 490-509 (1974)
2) Int. J. Syst. Bact. 16, 114 (1966)
3) Microbiol. Immunol. 25 (3), 257-269 (1981)
4) J. Clin. Pathol. 33, 53-57 (1980)
5) J. General Microbiol. 128, 713-720 (1982)
6) Applied Microbiol. 23 (6), 1131-1139 (1972)

The typical microbiological characteristics of the above-exemplified strains according to the present invention are summarized in Table 2.

3

## Table 2

| Characteristics | FERM BP -296 | -297 | -298 | -299 | -300 | -301 | -302 |
|---|---|---|---|---|---|---|---|
| Shape of cell | spheroid | | | | | | |
| Gram stain | + | + | + | + | + | + | + |
| Hemolysis | α | α | α | α | α | α | α |
| Growth at 10°C | + | + | ± | − | + | − | − |
| 45°C | + | + | + | ± | + | ± | + |
| 50°C | + | − | − | − | + | − | − |
| Thermal resistance at 60°C for 30 min | + | + | + | − | + | − | − |
| Growth in culture medium at pH 9.6 | + | + | + | − | + | − | − |
| Methylene blue reduction | + | + | − | − | + | − | − |
| Liquefaction of gelatin | − | − | − | − | − | − | − |
| Growth in culture medium containing NaCl (6.5%) | + | + | − | − | + | − | − |
| Growth in culture medium containing bile (40%) | + | + | + | − | + | − | + |
| Productivity of ammonia | + | + | ND[*2] | − | + | ± | − |
| Hydrolysis of hippuric acid | − | ± | − | − | + | − | − |
| Growth in culture medium containing tellurite | − | + | − | ND | − | ND | − |
| Growth in culture medium containing TTC[*1] | − | + | − | ND | − | ND | − |
| Acid production from | | | | | | | |
| Glucose | + | + | + | + | + | + | + |
| Esculin | ± | + | + | + | ± | ND | + |
| Inulin | − | − | − | + | − | − | ± |
| Lactose | + | + | + | ± | + | ± | − |
| Glycerol | − | + | ± | − | − | − | − |
| Arabinose | + | − | + | − | − | − | − |
| Melezitose | − | + | ± | ND | − | ND | − |
| Sorbitol | − | + | + | − | − | − | − |
| Antigenic group | D | D | Q(D) | K | D | − | D |

*1: 2,3,5-Triphenyltetrazolium chloride    *2: Not done .

3. Cultivating Methods

These microorganisms can be cultivated in a conventional manner. For example, the bacterial cells can be collected by stationary cultivation in a Rogosa broth medium (Efthymiou, C., and Hausen, P.A. (1962) An antigenic analysis of Lactobacillus acidophilus. J. Infect. Dis. 110: 258-267) having the following composition under an aerobical condition, and can be harvested by centrifugation of the culture.

## Composition of Rogosa broth medium

| | |
|---|---|
| Trypticase | 10 g |
| Yeast extract | 5 g |
| Tryptose | 3 g |
| $K_2HPO_4$ | 3 g |
| $KH_2PO_4$ | 3 g |
| Triammonium citrate | 2 g |
| Tween 80 | 1 g |
| Glucose | 20 g |
| Cysteine hydrochloride | 0.2 g |
| Salt solution *1 | 5 ml |
| Distilled water | to 1 liter |

(pH 7, heat sterilization at 121°C for 15 minutes)

| | | |
|---|---|---|
| *1 | $MgSO_4-7H_2O$ | 11.5 g |
| | $FeSO_4-7H_2O$ | 0.68 g |
| | $MnSO_4-2H_2O$ | 2.4 g |
| | Distilled water | 100 ml |

The resultant cells can be directly used as a pharmaceutical agent in the form of living cells or dead cells obtained by, for example, heat-treatment, or in the form of the cells destroyed by, for example, ultrasonic treatment. The term "dead cells" used herein means the entire portions or partial portions of the above-mentioned destructed cells.

The dead cells can be also used as a starting material for fractionating, extracting, and purifying active elements contained therein. This is because the desired pharmacological activities (i.e., liver function-improving and blood pressure-lowering activities in mammals) are based on substance components contained in the cells, and because not only the living cells but also the dead cells have these pharmacological activities.

Preparation of Bacterial Cells

The living cells and dead cells of the microorganisms used in the present invention, suitable for use as a liver function-improving agent and a blood pressure-lowering agent, are typically prepared as follows:

1. Preparation Example of the Living Cells

Each strain of the above-mentioned microorganisms is inoculated into 5 liters of the above-mentioned Rogosa broth medium and then stationarily cultivated under an aerobic condition at 37°C for 5 hours to yield the subsequent culture broth containing $10^9$/ml of the living cells. The microorganisms are harvested by continuous centrifugation at 12,000 rpm. The bacterial cells are washed twice with physiological saline and are then suspended in physiological saline to obtain 50 ml of the cell suspension containing $10^{11}$ cells/ml.

2. Preparation Example of the Dead Cells (the Heat-treated Cells)

Fifty ml of the living cell suspension containing $10^{11}$ cells/ml obtained as mentioned in the above Example was heated at 115 - 121 °C for 10 - 15 minutes to form the desired cell suspension obtaining the dead cells.

Pharmacological Actions

1. Pharmacological Effects

a) As shown in each example hereinbelow, the bacterial cell products obtained from the above-mentioned microorganisms of the present invention can achieve an extremely effective reduction of serum GOT and GPT levels in mammals as the liver function-improving agent, and have a strong effect in the lowering of high blood pressure to the normal range as the blood pressure-lowering agent. Accordingly, the bacterial cell products according to the present invention are useful as a therapeutical or preventive medicine for diseases to which these parameters of the serum and the high blood pressure closely related, such as not only myocardial infarction, cerebral apoplexy, atherosclerosis, but also acute or chronic hepatitis, cirrhosis, fatty liver, alcoholic hepatitis, hepatic tumor, bile stasis, muscle disease, hemolytic disease, disease of skeletal muscle, hypertension, and others.

The pharmaceutical composition according to the present invention can be generally applied in a dosage of $10^7$ to $10^{14}$ cells/kg body weight, more desirably $10^9$ to $10^{12}$ cells/kg body weight, by, for example, an oral administration. The pharmaceutical composition according to the present invention can be in the form of, for example, suspensions in physiological saline solutions, lyophilized powder, granules, tablets, and capsules, etc. The pharmaceutical composition according to the present invention can be optionally prepared by using conventional appropriate carriers, bulk fillers, diluents, and so on.

2. Acute Toxicity

As shown in the examples hereinbelow, as LD50 of the living cells according to the present invention is $8.9 \times 10^8$ to $1.3 \times 10^{10}$ cells/mouse, intraperitoneally and that of the dead cells according to the present invention is more than $6 \times 10^{11}$ cells/mouse, intraperitoneally.

Both the living and dead cells according to the present invention are substantially nontoxic upon oral administration.

Examples

The present invention will now be further shown by, but is by no means limited to, the following examples.

Example 1

Pharmacological Actions in Liver Functions (1)

The heat-treated cell suspension of Streptococcus faecalis ADV 9001 (FERM BP-297) prepared according to the above-mentioned dead (heat-treated) cell preparation method was lyophilized. The lyophilized sample obtained above was put into gelatin capsules and orally administered at a daily dosage of 60 mg to rabbits (New Zealand White rabbits, male, average body weight of 2 kg, Clea Japan, Inc. 5 rabbits in each group). Empty capsules were administered to the control group to which no sample was dosed.

Venous blood was taken from the auricular vein of the animals at 6, 7, 8, and 9 weeks after the start of administration, and the serum GOT and GPT levels were determined by the GOT and GPT determination kits (Eiken Chemical Co., Ltd.).

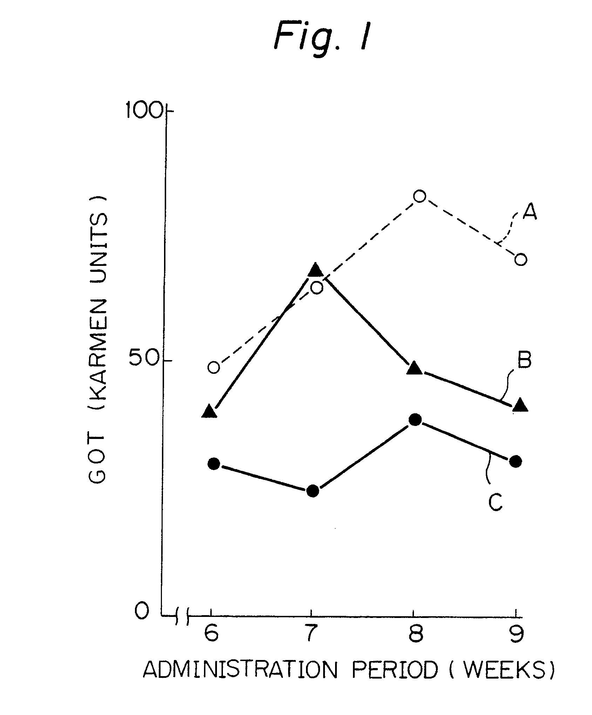

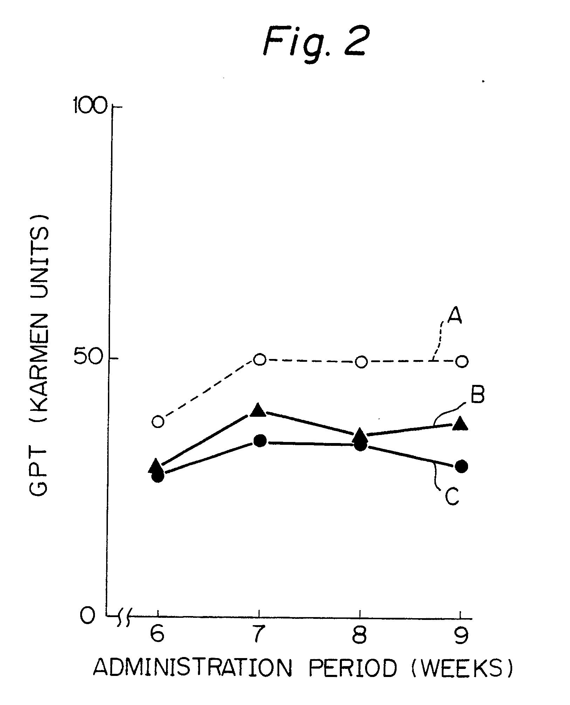

The results of the GOT and GPT levels (Karmen units) are shown in Figs. 1 and 2, respectively. In the figures, the ordinate and abscissa indicate the GOT or GPT level (Karmen units), and breeding period (weeks) after the start of administration, respectively, and A is the control group to which no sample and empty capsules were dosed, B is also a control group to which normal diet was administered, and C is an administration group with the heat-treated cells (60 mg/day).

The diet CR-1 (Clea Japan, Inc.) was given to the group B and the groups A and C were given the diet CR-1 containing 0.2% of cholesterol.

As is obvious from the figures, the administration of this agent according to the present invention keeps the GOT and GPT levels lower than 30 units, i.e., in the normal range.

Example 2

Pharmacological Actions in Liver Functions (2)

The freeze-dried materials of the heat-treated cell suspension of each microorganism belonging to the genus Streptococcus were obtained by the same method as in Example 1 and orally administered at a daily dosage of 60 mg into the rabbits (New Zealand White rabbits, male, average body weight of 2.01 kg, Clea Japan, Inc., 5 rabbits in each group).

The serum GOT and GPT levels (Karmen units) were then determined by the same method as in Example 1 and the results were shown in Table 3.

As is obvious from the table, the administration of this agent according to the present invention keeps the GOT and GPT levels at 20 - 40 units and 10 - 35 units, respectively.

## Table 3

| Microorganisms | | Breeding period (weeks) | | | |
|---|---|---|---|---|---|
| | | 6 | 7 | 8 | 9 |
| S. faecium | GOT | 23.1 | 28.6 | 29.2 | 25.3 |
| ADV1009 | GPT | 16.4 | 19.7 | 18.6 | 22.3 |
| S. avium | GOT | 30.5 | 28.1 | 28.9 | 33.5 |
| AD2003 | GPT | 29.4 | 32.6 | 24.3 | 31.1 |
| S. durans | GOT | 29.9 | 34.6 | 29.4 | 29.1 |
| ADV3001 | GPT | 25.5 | 31.5 | 31.9 | 32.4 |
| S. salivarius | GOT | 29.0 | 26.1 | 30.3 | 31.0 |
| ADV10001 | GPT | 20.3 | 29.4 | 30.1 | 31.9 |
| S. mitis | GOT | 34.5 | 30.1 | 31.6 | 29.6 |
| ADV7001 | GPT | 18.6 | 26.3 | 27.5 | 30.0 |
| S. equinus | GOT | 26.2 | 39.6 | 27.7 | 26.1 |
| ADV8001 | GPT | 29.9 | 31.6 | 28.7 | 31.8 |

Example 3

Pharmacological Actions in Blood Pressure (1)

After the preliminary breeding of conventional rats (Fischer 344, male, 5 week-old, average body weight of 156 g, Clea Japan, Inc., 5 rats in each group) on CE-II (Clea Japan, Inc.) ad libitum for 2 weeks, drinking water was changed to 1% NaCl solution. When the rats were 8 week-old, the freeze-dried material of the heat-treated cell suspension of Streptococcus ADV9001 (FERM BP-297) obtained by the same method as in Example 1 was orally administered at a daily dosage of 60 mg into the animals. The control group was not given the material. At the same time, measurement of the tail arterial blood pressure of the rats was started using a Type KN-210 Rat tail manometer-tachometer system (Natsume Seisakusho Co., Ltd.).

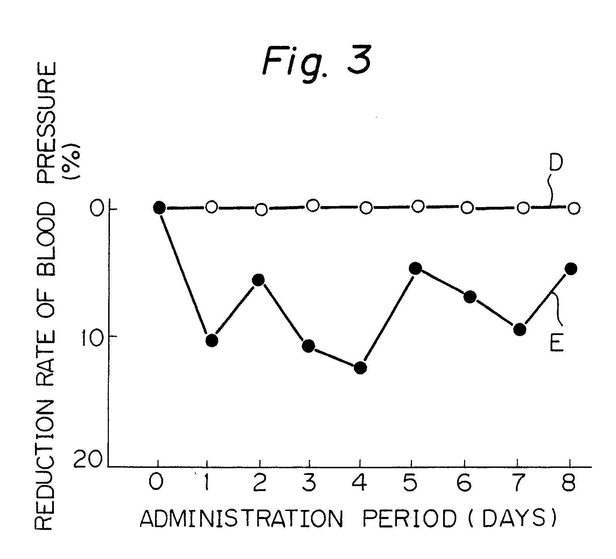

The reduction rate (%) was shown in Fig. 3. In the figure, the ordinate and abscissa indicate the reduction rate (%) of blood pressure and administration period (days), respectively, and D and E indicate the control and the administration group with the heat-treated cells, respectively. As is obvious from the figure, the reduction rate of the blood pressure in the animals is about 10% by administration of this agent according to the present invention.

Example 4

Pharmacological Actions in Blood Pressure (2)

The lyophilized materials of the heat-treated cell suspension of each microorganism belonging to the genus Streptococcus obtained by the same method as in Example 2 were administered into conventional rats (Fischer 344, 5 week-old males, average body weight of 162 g, Clea Japan, Inc., 5 rats in each group). The reduction rate (%) of the blood pressure was measured by the same method as shown in Example 3, and the results were shown in Table 4.

As is obvious from the table, the reduction rate of the blood pressure in the animals is brought to 7 - 13% by the administration of this agent according to the present invention.

### Table 4

| Microorganisms | Administration period (days) | | | | | | | | |
|---|---|---|---|---|---|---|---|---|---|
| | 0 | 1 | 2 | 3 | 4 | 5 | 6 | 7 | 8 |
| S. faecium ADV 1009 | 0 | 10.6 | 7.1 | 9.4 | 11.7 | 9.1 | 9.3 | 9.4 | 9.1 |
| S. avium AD2003 | 0 | 8.4 | 12.3 | 11.6 | 7.6 | 10.8 | 6.5 | 7.4 | 8.3 |
| S. durans ADV3001 | 0 | 4.3 | 6.3 | 7.8 | 7.6 | 10.4 | 7.1 | 6.8 | 6.9 |
| S. salivarius ADV10001 | 0 | 7.6 | 9.6 | 8.1 | 7.3 | 12.3 | 9.6 | 8.9 | 8.1 |
| S. mitis ADV7001 | 0 | 8.1 | 7.4 | 10.0 | 8.1 | 7.3 | 10.3 | 8.2 | 7.3 |
| S. equinus ADV8001 | 0 | 6.3 | 10.3 | 9.4 | 7.2 | 6.9 | 10.1 | 7.1 | 11.6 |

Example 5

Living cells of bacteria prepared according to the above-mentioned living cell preparation method were intraperitoneally administered to ICR mice (6 week-old males, average body weight of $30.0 \pm 0.7$ g) with 0.5 ml of a bacterial suspension containing $9 \times 10^9$ $9 \times 10^8$ and $9 \times 10^7$ cells per mouse (10 mice in each group). Thanatobiologic observation of the mice was carried out for 14 days.

The LD50 values (viable cell number/mouse) calculated according to a Behrens-Kärber method are shown in Table 5.

In the case of the dead cells of the above-mentioned strains according to the present invention, the LD50 values corresponded to more than $6 \times 10^{11}$ cells/mouse (intraperitoneal dosage). Both the living and dead cells of the above-mentioned strains according to the present invention were nontoxic in the case of oral administration.

## Table 5

| Strains | LD50 (viable cell number/mouse) |
|---------|--------------------------------|
| S. faecium ADV1009 | $6.3 \times 10^9$ |
| S. faecalis ADV9001 | $3.8 \times 10^9$ |
| S. avium AD2003 | $4.2 \times 10^9$ |
| S. durans ADV3001 | $8.9 \times 10^9$ |

Example 6 (Pharmaceutical preparations)

1. A 50 mg amount (corresponding to $5 \times 10^{10}$ cells) of freeze dried powder of S. faecium ADV1009 living cells prepared according to the above-mentioned living cell preparation method was uniformly mixed with 950 mg of purified starch powder and then tablets were formed for oral administration. Each tablet corresponds to a dosage of $10^9$ cells/kg body weight for a human adult having a body weight of 50 kg.

2. A tablet, obtained from 500 mg of the above-mentioned freeze-dried powder by mixing with 500 mg of purified starch powder, corresponds to a dosage of $10^{10}$ cells/kg body weight.

Thus, the cell products of the present invention can be converted into the desired dosage form having a predetermined activity by mixing with pharmaceutically acceptable carriers based on the above-mentioned standard dosage.

## Claims

1. Use of a microorganism belonging to the genus Streptococcus in the preparation of a pharmaceutical composition for lowering serum GOT and GPT levels, or lowering blood pressure.

2. Use of a microorganism comprising at least one member selected from S. faecium, S. faecalis, S. avium, S. durans, S. salivarius, S. mitis, and/or S. equinus in the preparation of a pharmaceutical composition for controlling serum GOT and GPT levels, or lowering blood pressure.

## Revendications

1. Utilisation d'un micro-organisme appartenant au genre Streptococcus dans la préparation d'une composition pharmaceutique propre à réduire les taux sériques de GOT et de GPT ou à abaisser la pression sanguine.

2. Utilisation d'un micro-organisme comprenant au moins un membre choisi parmi S. faecium, S. faecalis, S. avium, S. durans, S. salivarius, S. mitis et/ou S. equinus dans la préparation d'une composition pharmaceutique propre à contrôler les taux sériques de GOT et de GPT ou à abaisser la pression sanguine.

## Patentansprüche

1. Verwendung eines Mikroorganismus der Art Streptococcus bei der Herstellung einer pharmazeutischen Zubereitung zur Verminderung des GOT- und des GPT-Serumspiegels oder zur Verminderung des Blutdrucks.

2. Verwendung eines Mikroorganismus umfassend mindestens ein Mitglied von S. faecium, S. faecalis, S. avium, S. durans, S. salivarius, S. mitis, and/or S. equinus bei der Herstellung einer pharmazeutischen Zubereitung zur Kontrolle des GOT- und des GPT-Serumspiegels oder zur Verminderung des Blutdrucks.

# Fig. 1

## Fig. 2

Fig. 3